# EUROPEAN PATENT SPECIFICATION

(11) **EP 4 464 310 B1**
(45) Date of publication and mention of the grant of the patent: **14.10.2026**
(21) Application number: 24190177.6
(22) Date of filing: 01.04.2015
(51) Int. Cl.: A61L 27/54, A61F 2/04, A61K 9/16, A61K 9/00, A61K 45/00, A61K 47/12, A61K 47/18, A61K 47/34, A61F 6/14, A61M 31/00, A61K 47/20

(54) **CONTRACEPTIVE AND RELATED DEVICE**
EMPFÄNGNISVERHÜTENDES MITTEL UND ZUGEHÖRIGE VORRICHTUNG
CONTRACEPTIF ET DISPOSITIF ASSOCIE

(30) Priority: 01.04.2014 US 201461973816 P
(43) Date of publication of application: 20.11.2024
(62) Document number(s) of the earlier application(s) in accordance with Art. 76 EPC: 15774399.8 / 3 125 964
(73) Proprietor: Poly-Med, Inc., Anderson, SC 29625 (US)
(72) Inventor: GRAY, Kenneth David Jr, Columbia, 29209 (US); VAUGHN, Michael Aaron, Clemson, 29631 (US); HILAS, Georgios Theofanis, Bloomington, 47408 (US)
(74) Representative: Uexküll & Stolberg

(56) References cited:
- WO-A1-2013/013172
- US-A1- 2004 265 355
- US-A1- 2005 053 639
- US-A1- 2008 069 850

## Description

### CROSS-REFERENCE TO RELATED APPLICATION

This application claims the benefit under 35 U.S.C. § 119(e) of U.S. Provisional Patent Application No. 61/973,816 filed April 1, 2014.

### FIELD OF THE INVENTION

The present invention relates generally to intravaginal rings and to the release of bioactive agents therefrom.

### BACKGROUND

In recent years, a variety of women's health issues have generated considerable interest. Areas of particular importance have included obstetrics, medical terminations, contraception, infertility, sexually transmitted infections, and cancers of the reproductive tract. In the postmenopausal female, the reduction of endogenous estrogen has been shown to profoundly influence the skeletal and lower urogenital systems. Concurrently, a number of therapeutic strategies have been developed to improve treatment in these respective areas. While oral, intravenous, and transdermal routes of drug administration have been widely utilized, intravaginal drug release has been studied to a far lesser extent. Interestingly, there are many instances where intravaginal drug release may by ideal. For example, it is postulated that drug specificity for the reproductive tract may be more effectively achieved through intravaginal administration. Thus, elevated levels may be attained at a fraction of the oral or parenteral doses. A secondary benefit would be to improve patient compliance in terms of dosing frequency and/or systemic side effects. The rich vascular supply of the vagina also represents a rapid portal of entry when systemic drug levels are desired. Because of the anatomy, first-pass hepatic metabolism is bypassed which could be utilized to improve the relative bioavailability of certain agents.

Intravaginal drug release can be utilized for topical, local, or systemic effects. Topical administration has been used in the treatment of bacterial or fungal infections, atrophic vaginitis, and vaginal intraepithelial neoplasia. In terms of local therapy, vaginal drug administration has been used to treat stress urinary incontinence, labor induction, medical abortions, and infertility. The advantage of this route is the large surface area for drug absorption and ease of administration. US2008/069850A1 discloses a multicomponent microbicidal contraceptive intravaginal ringed-mesh device comprising a ringed-mesh construct comprising a ring matrix encircling a fibrous mesh adjoined thereto and held in the ring lumen and a polymeric coating covering the ringed-mesh construct, wherein each of the ring matrix and the polymeric coating comprise at least one drug-releasing polymeric substrate for the independent controlled release of at least one contraceptive agent and at least one bioactive agent.

### SUMMARY

The present disclosure provides devices for intravaginal release of bioactive agents, as defined by the claims. Briefly stated, an intravaginal ring for the controlled release of at least one bioactive agent, as defined by the claims, comprises a biostable elastomeric porous polymeric matrix, to aid in the release and/or modulate the release of the bioactive agent.

For example, the present disclosure provides a partially absorbable composite for the controlled release of at least one bioactive agent comprising a biostable, elastomeric polymeric matrix, the matrix further containing an absorbable microparticulate ion-exchanging polymer to modulate the release of the bioactive agent for a desired period of time at a specific biological site. As another example, the present disclosure provides an intravaginal ring for the controlled release of at least one bioactive agent comprising a biostable, elastomeric polymeric matrix, the matrix further containing an absorbable microparticulate ion-exchanging polymer to modulate the release of the bioactive agent for a desired period of time at a specific biological site. In another aspect, the present disclosure provides an intravaginal ring for the controlled release of at least one bioactive agent comprising a biostable, elastomeric polymeric matrix, the matrix further containing an essentially non-bioabsorbable microparticulate ion-exchanging polymer to modulate the release of the bioactive agent for a desired period of time at a specific biological site.

In yet another aspect, the present disclosure provides an intravaginal ring for the controlled release of at least one bioactive agent comprising a fully bioabsorbable, polymeric matrix, the matrix further containing an absorbable microparticulate ion-exchanging polymer to modulate the release of the bioactive agent for a desired period of time at a specific biological site. In addition, the present disclosure provides an intravaginal ring for the controlled release of at least one bioactive agent comprising a biostable, hydrophilic elastomeric polymeric matrix, the matrix further containing an absorbable microparticulate ion-exchanging polymer to modulate the release of the bioactive agent for a desired period of time at a specific biological site. As another example, the present disclosure provides an intravaginal ring for the controlled release of at least one bioactive agent comprising a biostable, amphiphilic elastomeric polymeric matrix, the matrix further containing an absorbable microparticulate ion-exchanging polymer to modulate the release of the bioactive agent for a desired period of time at a specific biological site. Yet further, the present disclosure provides an intravaginal ring for the controlled release of at least one bioactive agent comprising a biostable, elastomeric polymeric matrix, the matrix further containing an inorganic microparticulate to modulate the release of the bioactive agent for a desired period of time at a specific biological site. As defined by the claims, the present invention provides an intravaginal ring for the controlled release of at least one bioactive agent, the ring comprising a) a polymeric matrix and b) an absorbable polymeric microparticulate to modulate the release of the bioactive agent, wherein the matrix becomes microporous as the microparticulate degrades in the biological environment over time. Also, the present disclosure provides an intravaginal ring for the controlled release of at least one bioactive agent comprising biostable, elastomeric porous polymeric matrix, the matrix further containing an absorbable microparticulate ion-exchanging polymer to modulate the release of the bioactive agent for a desired period of time at a specific biological site.

The details of one or more embodiments are set forth in the description below.

### DETAILED DESCRIPTION OF THE INVENTION

The present disclosure provides devices for implantation into a human body, e.g., contraceptive devices, and constructs that may be used to form such devices such as intravaginal rings. The present invention may be understood more readily by reference to the following detailed description including preferred embodiments of the invention and the Examples included herein. It is to be understood that the terminology used herein is for the purpose of describing specific embodiments only and is not intended to be limiting. It is further to be understood that unless specifically defined herein, the terminology used herein is to be given its traditional meaning as known in the relevant art. Headings used within this document are only being utilized to expedite its review by the reader, and should not be construed as limiting the invention or claims in any manner.

As used throughout this document, including the claims, the singular form "a", "an", and "the" include plural references unless indicated otherwise. For example, "a bioactive agent includes one or more bioactive agents. As another example, "a" microparticulate refers to one or more microparticulates. The following terms have the indicated meanings. Other terms used herein may be understood by reference to U.S. Patent Nos. 8,057,817 and 8,404,272.

Absorbable microparticulate ion-exchanging polymer refers to a bioabsorbable polymer, such as a polyester that degrades by hydrolysis. Such polyesters may be derived from cyclic monomers selected from the group consisting of lactides, glycolide, epsilon-caprolactone, trimethylene carbonate, and para-dioxanone, and combinations thereof. The polymer can be synthesized with acid end groups that provide the ion-exchanging character. For instance, glycolic acid can be used as an initiator during synthesis of low molecular weight polymers to provide the acid end groups. The bioabsorbable polyester is processed by grinding the material into a fine powder to create the microparticulates. US Patent No. 6,413,539 describes the synthesis of such polymers and their processing to create microparticulates. Examples include polyglycolide, poly(glycolide-co-trimethylene carbonate), polylactide, poly(glycolide-co-caprolactone). Absorbable microparticles that are not ion-exchanging may be prepared by initiating ring-opening polymerization with an alcohol, such as 1,3-propane diol or decyl alcohol. These initiators lack carboxylic acid groups, which would provide the ion-exchanging functionality.

Biostable amphiphilic elastomeric polymeric matrix refers to a non-absorbable polymeric construct that serves as a reservoir for release of a bioactive agent or agents. The polymeric matrix is constructed from an amphiphilic copolymer that contains hydrophilic and hydrophobic chain segments or blocks, such that the copolymer can form separate domains that either absorb water or repel water. Similarly, these domains contain either polar or nonpolar bioactive agents. The polar bioactive agents will typically segregate to the hydrophilic domains, and the nonpolar bioactive agents will typically segregate to the hydrophobic domains. The addition of hydrophilic segments has the advantage of potentially modifying the release rate of bioactive agents from the matrix by increasing water absorption. Furthermore, the copolymers suitable for use in this application are designed to be elastomeric, thereby creating a final device that is flexible and compliant. This elastomeric character facilitates with insertion of the device and is intended to improve overall comfort. Examples of such polymers include poly(dimethyl siloxane)-containing block copolymers, poly(dimethyl siloxane)-block-poly(ethylene glycol), poly(dimethyl siloxane)-block-poly(vinyl alcohol), poly(dimethyl siloxane)-block-poly(acrylic acid), poly(2-hydroxyethyl methacrylate-g-dimethylsiloxane), poly(2,3-dihydroxypropyl methacrylate-g-dimethylsiloxane), poly(dimethylacrylamide)-block-poly(dimethyl siloxane)-block-poly(dimethylacrylamide, poly(dimethyl siloxane)-block-poly(2-(dimethylamino)ethyl acrylate). The foregoing polymers can be described differently, for instance as block copolymers of PDMS and a second hydrophilic component, such as those listed here.

Biostable elastomeric polymeric matrix refers to a non-absorbable porous polymeric construct that serves as a reservoir for release of a bioactive agent or agents. The matrix is constructed from a material that is stable in the biological environment and does not significantly degrade in an aqueous environment or in the presence of enzymes during the period of implantation. Furthermore, the matrix contains a porous microstructure, preferably an open-pore structure that allows for the influx of aqueous fluid from the surrounding environment and facilitates the release of bioactive agents from the construct. The polymers suitable for this type of matrix are elastomers that provide flexibility and compliancy, and the polymers can be tailored to provide the desired water absorption. Examples include silicone-based polymers, polyurethanes, polyolefins, polydienes, poly(ethylene-co-vinyl acetate), and cross-linked versions of these polymers.

Biostable hydrophilic elastomeric polymeric matrix refers to a non-absorbable polymeric construct that serves as a reservoir for delivery of a bioactive agent or agents. The polymeric matrix is constructed from a hydrophilic polymer or copolymer, or alternatively from a polymer or copolymer that is blended with hydrophilic additives that improve water absorption. The hydrophilic nature of the matrix is intended to improve the delivery of bioactive agents from the matrix to the surrounding biological environment by facilitating diffusion of bioactive agents through the matrix. In some embodiments, the absorption of water can cause the matrix to swell, which can increase void volume and thereby facilitate diffusion. Furthermore, a hydrophilic polymeric matrix is intended to be more effective than a relatively hydrophobic polymeric matrix at delivering bioactive agents, since the hydrophobic matrix may retain bioactive agents and demonstrate slow rates of diffusion. Examples of materials from which to form a device having a biostable hydrophilic elastomeric polymeric matrix include: ethylene-vinyl acetate, poly(dimethyl siloxane)-block-poly(ethylene glycol), poly(dimethyl siloxane)-block-poly(vinyl alcohol), poly(dimethyl siloxane)-block-poly(acrylic acid), poly(2-hydroxyethyl methacrylate-g-dimethylsiloxane), poly(2,3-dihydroxypropyl methacrylate-g-dimethylsiloxane), poly(dimethylacrylamide)-block-poly(dimethyl siloxane)-block-poly(dimethylacrylamide, poly(dimethyl siloxane)-block-poly(2-(dimethylamino)ethyl acrylate). Other examples include the aforementioned polymers and copolymers blended with poly(ethylene glycol), poly(vinyl alcohol), copolymers of poly(ethylene glycol) and poly(propylene glycol), poly(acrylic acid), and mixtures thereof.

Essentially non-bioabsorbable microparticulate ion-exchanging polymer refers to a polyester that is derived from cyclic monomers selected from the group consisting of lactides, glycolide, epsilon-caprolactone, trimethylene carbonate, and para-dioxanone, and combinations thereof. The polymer is essentially stable and does not degrade during the time frame of implantation, and is therefore described as essentially non-bioabsorbable. Additionally, the polyester can be synthesized with acid end groups that provide ion-exchanging character. For instance, glycolic acid can be used as an initiator during synthesis of low molecular weight polymers to provide the acid end groups. The bioabsorbable polyester is processed by grinding the material into a fine powder to create the microparticulates. US Patent No. 6,413,539 describes the synthesis of such polymers and their processing to create microparticulates. Examples include polylactide, poly(lactide-co-trimethylene carbonate), poly(lactide-co-caprolactone).

Fiber reinforcement and a fiber construct refers to the incorporation of fiber into the device, construct or ring of the present disclosure. The incorporation of a fiber, either an absorbable or nonabsorbable fiber, can provide one or more benefits, including imparting increased overall stiffness, providing structural support, and/or maintaining the original dimensions of the polymeric construct. The fiber can be fabricated from absorbable materials, such as copolymers or homopolymers of lactides, glycolide, caprolactone, p-dioxanone, and trimethylene carbonate, or alternatively, the fiber can be fabricated from nonabsorbable materials, such as nylon, polypropylene, polyethylene terephthalate, or from metallic fibers or filaments, such as copper, iron, tungsten, and various alloys of these and other metals. Metallic fibers and filaments typically provide greater strength and stiffness, which allows such materials to help maintain the original shape of the construct. The reinforcing fibers described here can be utilized in different forms, exemplary forms being (1) a reinforcing fibrous ring embedded within the interior of a polymeric ring-shaped construct, (2) fibers dispersed and embedded randomly throughout a polymeric ring-shaped construct, or (3) fibers wrapped in a spiral formation around the exterior of a polymeric ring-shaped construct.

As additional examples, the fiber may be (1) made of a continuous multifilament or monofilament yam of an absorbable, biodegradable polymer with intrinsic or engineering modulus in the moderate or high range; (2) twisted chitosan staples which may be treated with an absorbable synthetic coating to increase its engineering modulus; (3) based on a polyester or copolyester which is derived from one or more of the following monomer(s): glycolide, I-lactide, dl-lactide, trimethylene carbonate, p-dioxanone, .epsilon.-caprolactone, morpholinedione; (4) based on a segmented or block copolymer made by end-grafting polyalkylene dicarboxylate, such as polyethylene succinate, with one or more of the cyclic monomer(s) of item 3; and (5) made of twisted yarn, braid, twisted/coated staples, or non-woven fabric in the form of a ring structure. A fiber may be chemically treated primarily at its surface to create basic or acidic groups for binding a bioactive agent, such as an ionic, acidic or basic drug, for providing an additional mode for controlling the release of an agent other than simple diffusion through the matrix or through physical liberation as the matrix degrades, as in the case of absorbable/biodegradable matrices. The use of fibers provides for fiber reinforcement and a fiber-reinforced composite.

Non-absorbable matrix refers to a material, typically a polymeric material, that may be used to form a device of the present disclosure, and which does not completely degrade and become absorbed into the host that receives the device. In other words, a device formed from a non-absorbable matrix will remain largely or entirely intact in the body for an extended period of time, e.g., at least one year. A useful polymeric material to form a non-absorbable matrix may be based on (1) poly dimethyl siloxane with or without aromatic sequences serving as a modifier and crosslinked siloxane-based system; and (2) a methacrylate polymer derived from one or more alkyl methacrylate(s) such as n-hexyl methacrylate, n-butyl methacrylate, with our without a more hydrophilic monomer such as vinyl acetate and/or or N-vinyl pyrrolidone.

Polymeric microparticles refer to small particles that are made from organic polymers. In one embodiment, the microparticles have an average diameter in the micron range, i.e., in the range of 1 to 100 microns. In another embodiment, the microparticles have micron and sub-micron dimensions ranging from 0.5 µm to 100 µm, preferably 10 µm to 80 µm and more preferably 20 µm to 70 µm. In one embodiment, absorbable polymeric microparticulate refers to a bioabsorbable polyester that degrades by hydrolysis and is derived from cyclic monomers selected from the group consisting of lactides, glycolide, epsilon-caprolactone, trimethylene carbonate, and para-dioxanone, and combinations thereof. The polymer can be synthesized using mono- or multifunctional initiators to create linear or multi-axial polymers. Microparticulates may be created from these bulk polymers by grinding the synthesized material into a fine powder and then using a sieve of appropriate mesh size to isolate the desired sized particles. Examples of organic polymers include polyglycolide, polylactide, polycaprolactone, poly(para-dioxanone), poly(glycolide-co-trimethylene carbonate), poly(glycolide-co-caprolactone), and poly(glycolide-co-L-lactide). For instance, glycolide may be polymerized in the presence of glycolic acid and stannous octanoate to produce low molecular weight, hydrolytically degradable polyester as described in U.S. Pat. No. 6,413,539. Purification and reduction in size of the polyester may also be conducted as per the description in U.S. Pat. No. 6,413,539 to provide an acid-terminated polyglycolide microparticulate.

As additional examples, microparticles may be made of a lactide based polymer or a solid semi-crystalline polylactone such as polyglycolide which can be formed by ring opening polymerization of acid-bearing hydroxylic initiators such as glycolic, lactic, malic, tartaric, and citric acid. A microparticle can be synthesized according to the following procedure. In a reaction vessel are mixed a lactide based monomer and/or a lactone such as glycolide and an acid initiator such as tartaric acid, malic acid or citric acid. The reaction vessel is warmed to about 35-45C, preferably 40C and put under vacuum for about 20-60 minutes, preferably 30 minutes. The temperature of the reaction vessel is raised to about 105-115C, preferably 110C. Once this temperature is reached the vessel is placed under an atmosphere of oxygen-free nitrogen, and the mixture is stirred. Once the mixture melts, a catalytic amount of an organometallic catalyst suitable for ring opening polymerization, such as stannous 2-ethyl-hexanoate solution in a non-protic solvent, such as toluene is added. A vacuum is reapplied for about 30-90 seconds to remove toluene without significant removal of monomer. The temperature of the mixture is raised to about 115-125C, preferably 120C for about 5-10 minutes before further raising it to about 145-150C. It was kept at this temperature for about 3-5 hours, preferably 4 hours, under constant mechanical stirring, if possible. The resulting polymer is micronized by initially grinding it using a Knife-grinder. The polymer is then micronized in an Aljet Micronizer using a pressurized dry nitrogen stream. The mean particle diameter size is analyzed in a Malvern Mastersizer/E using a volume distribution model and 200/5 cS silicone oil as dispersant."

Pre-determined period of time refers a release of bioactive agent from a device or ring or construct of the present disclosure, over a time course during which the bioactive agent is biologically effective to achieve its intended purpose.

In one aspect, the present disclosure provides contraceptive and related devices such as vaginal rings, and compositions from which to prepare such rings and related constructs useful in the preparation of contraceptive devices. An exemplary contraceptive device is a vaginal ring, wherein the vaginal ring may be in the form of a tubular-shaped material having a circular cross-section. The shape of the ring may vary from a perfect circle to an ellipse to practically a ribbon, depending on the composition and physical properties of the matrix and reinforcing fibers. When the ring is formed from a tubular material, the average diameter of the ring material may vary from about 1 to 25 mm.

The contraceptive device may contain one or more bioactive agents. Bioactive agent refers to chemicals (e.g., small molecules, peptides, proteins) that are useful for contraception, labor induction, intravaginal and transvaginal prevention or treatment of bacterial, fungal, viral or parasitic infection, cervical cancer, and ovarian cancer. The incorporation of a bioactive agent into a device of the present disclosure may provide for hormone replacement therapy, achieving contraception, treating infertility, managing infectious diseases, and use in gynecological cancer. The device or construct may optionally comprise an antifertility drug, such as testosterone and testosterone precursor, a spermicidal agent, or sperm immobilizer, and bisphosphonate. Additionally, the device or construct can be used for the controlled release of drugs having antiprogestinic anesthetic, analgesic, anti-inflammatory, antimicrobial, antiviral, or antipsychotic properties. The intravaginal ring can also be used for the controlled release of antibodies especially the monoclonal types, immunomodulator vaccines especially the recombinant types, and hematopoietic growth factors. The bioactive agent may be an ionic conjugate of a basic antimicrobial drug having lower solubility in the polymeric matrix than the free-basic drug, and wherein the basic drug is selected from the group represented by metronidazole and miconazole and the acidic component of the conjugate is pamoic acid or its monosodium salt.

In one embodiment, the device contains between 0.0001% to 40% of its weight of bioactive agent(s); and (2) is designed to release at least one bioactive agent for providing at least one of hormone replacement therapy, achieving contraception, treating infertility, managing infectious diseases, and use in gynecologic oncology. For example, the device may contain natural or synthetic estrogens and progestational agents for contraception, micronized progesterone or LH-releasing hormone and its synthetic analogs for infertility, prostaglandin analogs for labor induction/augmentation, somatostatin or its synthetic analogs, antineoplastic/angiogenic drugs such as paclitaxel, cisplatin, 5-FU, and curcumin, non-steroidal anti-inflammatory drugs such as naproxen, immunomodulating agents, antibiotic and anti-mycotic agents, spermicidal agents, and virucidal agents. The device may provide a more effective controlled release system than most orally, transdermally, inhalable, injectable drugs that are commonly are used for (1) relieving headache; (2) treating allergy; (3) treating the common cold; (4) treating cervical or uterine cancer; (5) treating flue infection; (6) treating human immunodeficiency virus (HIV); (7) treating different forms of bacterial, fungal, and viral infections, particularly those pertaining to the female genital system; (8) administering spermicidal agents or sperm immobilizer drugs having anesthetic, analgesic, antipyretic, antiprogestinic, and antipsychotic properties. The device may be used for the release of antibodies, especially the monoclonal types of immunomodulators, vaccines especially the recombinant types, insulin, and hematopoietic growth factor. The device may be used to deliver bioactive agents for (1) facilitating labor induction or controlled abortion; (2) treatment of intravaginal or transvaginal bacterial, fungal, viral, or parasitic infections; and/or (3) treating osteoporosis and especially those based on bisphosphonates.

In one aspect, the present invention provides a partially absorbable composite for the controlled release of at least one bioactive agent. The composite comprises a biostable, elastomeric polymeric matrix as defined by the claims. The matrix comprises an absorbable microparticulate polymer which is effective to modulate the release of the bioactive agent from the composite for a pre-determined period of time at a specific biological site. The composition is not fiber reinforced.

In one aspect, the present disclosure provides an intravaginal ring for the controlled release of at least one bioactive agent. The intravaginal ring comprises a biostable, elastomeric polymeric matrix, as defined by the claims. The matrix comprises an absorbable microparticulate polymer which is effective to modulate the release of the bioactive agent from the ring for a desired period of time at a specific biological site. The intravaginal ring is not fiber reinforced.

In one aspect, the present disclosure provides an intravaginal ring for the controlled release of at least one bioactive agent. The intravaginal ring comprises a biostable, elastomeric polymeric matrix. The matrix comprises an essentially non-bioabsorbable microparticulate ion-exchanging polymer which is effective to modulate the release of the bioactive agent from the ring for a pre-determined period of time at a specific biological site. The intravaginal ring may be fiber reinforced, but in one embodiment the ring is not fiber reinforced.

In one aspect, the present disclosure provides an intravaginal ring for the controlled release of at least one bioactive agent. The intravaginal ring comprises a fully bioabsorbable, polymeric matrix. The matrix comprises an absorbable microparticulate ion-exchanging polymer which is effective to modulate the release of the bioactive agent from the ring for a desired period of time at a specific biological site. The intravaginal ring may be fiber reinforced, but in one embodiment the ring is not fiber reinforced.

In one aspect, the present disclosure provides an intravaginal ring for the controlled release of at least one bioactive agent. The intravaginal ring comprises a biostable hydrophilic elastomeric polymeric matrix. The matrix comprises an absorbable microparticulate ion-exchanging polymer which is effective to modulate the release of the bioactive agent from the ring for a pre-determined period of time at a specific biological site. The intravaginal ring may be fiber reinforced, but in one embodiment the ring is not fiber reinforced.

In one aspect, the present disclosure provides an intravaginal ring for the controlled release of at least one bioactive agent. The intravaginal ring comprises a biostable amphiphilic elastomeric polymeric matrix. The matrix comprises an absorbable microparticulate ion-exchanging polymer which is effective to modulate the release of the bioactive agent from the ring for a pre-determined period of time at a specific biological site. The intravaginal ring may be fiber reinforced, but in one embodiment the ring is not fiber reinforced.

In one aspect, the present disclosure provides an intravaginal ring for the controlled release of at least one bioactive agent. The intravaginal ring comprises a biostable elastomeric polymeric matrix. The matrix comprises an inorganic microparticulate effective to modulate the release of the bioactive agent from the intravaginal ring for a pre-determined period of time at a specific biological site. The intravaginal ring may be fiber reinforced, but in one embodiment the ring is not fiber reinforced.

As defined by the claims, the present invention provides an intravaginal ring for the controlled release of at least one bioactive agent. The intravaginal ring comprises a) a polymeric matrix and b) an absorbable polymeric microparticulate which is effective to modulate the release of the bioactive agent from the ring. The matrix becomes microporous as the microparticulate degrades, i.e., is absorbed into the biological environment over time.

In one aspect, the present invention provides an intravaginal ring for the controlled release of at least one bioactive agent. The intravaginal ring comprise a biostable, elastomeric porous polymeric matrix, as defined by the claims. The matrix comprises an absorbable microparticulate polymer effective to modulate the release of the bioactive agent from the ring for a pre-determined period of time at a specific biological site.

In one aspect, the present disclosure provides an intravaginal ring for the controlled release of at least one bioactive agent, optionally a non-hormonal bioactive agent. The intravaginal ring comprises a polymeric matrix. At least one bioactive agent may be a reducing agent which reduces the oxidation state of at least one other bioactive agent, where the at least one other bioactive agent provides a spermiostatic effect. In optional embodiments, one or more of the following may further describe this aspect of the present disclosure: the reducing agent is selected from the group of metal ions, organic compounds, inorganic salts, organometallic salts, organometallic complexes, peptides, and polymers; the reducing agent is a metal ion in the form of iron, copper, zinc, or cobalt; the reducing agent is in the form of an organic compound that is selected from cysteine, N-acetyl-cysteine, thiol-functionalized amino acids, cysteine-containing peptides, glutathione, oxalic acid, D-ascorbic acid, tocopherols, tocotrienols, α-tocopherol, gamma-tocopherol, and phenols; the reducing agent is an inorganic salt that is selected from zinc carbonate, zinc chloride, zinc sulfide, copper sulfate, copper chloride, and iron chloride, iron carbonate, and iron sulfate; the reducing agent is an organometallic salt selected from zinc gluconate, zinc methyl, zinc ethyl, and copper gluconate; the reducing agent is in the form of a peptide containing at least one cysteine amino acid; the reducing agent is in the form of a polymer that is selected from synthetic polymers, naturally occurring polymers, chemically modified naturally occurring polymers; the reducing agent is in the form of a polymer selected from polyphenols, synthetic polymers functionalized with thiol groups, thiolated polyethylene glycol, polypeptides containing cysteine amino acids and biopolymers functionalized with thiol groups; the reducing agent is in the form of a synthetic polymer functionalized with thiol groups, for example, thiolated chitosan and thiolated hyaluronic acid; the reducing agent is in the form of a thiolated polyethylene glycol where optionally the polyethylene glycol is at least one of linear, branched, cross-linked, or has a star configuration, and optionally the thiol groups are present at the polymer chain ends; and optionally the thiol groups are present as side groups along the polymer chain; and optionally the thiol groups are present at the polymer chain ends and as side groups along the polymer chain; the at least one bioactive agent comprises at least two different reducing metals; the polymer matrix comprises additives that are capable of stabilizing the oxidation sate of reducing metals present within the polymer matrix, where optionally the additive is a salt of a non-reducing metal with chloride, carbonate, sulfate, or gluconate counter ions; the at least one other bioactive agent provides a spermiostatic effect through lipid peroxidation of sperm cell membranes; the at least one other bioactive agent is intended for use as a contraceptive and provides a spermiostatic effect through lipid peroxidation of sperm cell membranes.

In another aspect, not according to the invention, the present disclosure provides a partially absorbable, fiber-reinforced composite for the controlled release of at least one bioactive agent. The composite comprises an absorbable fiber construct located within a biostable elastomeric copolymeric matrix. The matrix comprises multiple layers effective to modulate the release of the bioactive agent from the composition for a pre-determined period of time at a specific biological site. In optional embodiments, one or more of the following may further describe this aspect of the present disclosure: the composite is an intravaginal ring; the multiple layers are of the same composition; the multiple layers are of different compositions; the multiple layers each contain the same bioactive agent(s); the bioactive agent(s) is present in different concentrations within at least two of the multiple layers; the multiple layers contain the same bioactive agent; the multiple layers each contain a different bioactive agent.

In another aspect, the present disclosure provides a partially absorbable, fiber-reinforced composite for the controlled release of at least one bioactive agent. The composite comprises an absorbable fiber construct located within a biostable elastomeric, copolymeric matrix. The matrix comprises multiple layers. The matrix comprises an absorbable microparticulate ion-exchanging polymer within at least one of the multiple layers, the ion-exchanging polymer being effective to modulate the release of the bioactive agent from the composite for a pre-determined period of time at a specific biological site. In optional embodiments, one or more of the following may further describe this aspect of the present disclosure: the multiple layers function to modulate the release of the bioactive agent; the absorbable microparticulate ion-exchanging polymer is located in only one of the multiple layers; the absorbable microparticulate ion-exchanging polymer is located in more than one of the multiple layers; the multiple layers comprise an innermost layer and an outermost layer, and the absorbable microparticulate ion-exchanging polymer is located within the innermost layer; the multiple layers comprise an innermost layer and an outermost layer, and the absorbable microparticulate ion-exchanging polymer is located within the outermost layer; the matrix comprises multiple layers and each of the multiple layers comprises the same bioactive agent(s); the bioactive agent(s) are present in different concentrations within each of the multiple layer; the matrix comprises multiple layers where each layer comprises the same bioactive agent(s); the matrix comprises multiple layers where each layer comprises a different bioactive agent(s).

In another aspect, the present disclosure provides a partially absorbable, fiber-reinforced composite for the controlled release of at least one bioactive agent. The composite comprises an absorbable fiber construct located within a biostable elastomeric amphiphilic copolymeric matrix. The matrix comprises an absorbable microparticulate ion-exchanging polymer which is effective to modulate the release of the bioactive agent for a pre-determined period of time at a specific biological site. In optional embodiments, one or more of the following may further describe this aspect of the present disclosure: the amphiphilic copolymeric matrix comprises polymer segments selected from polyethylene glycol, polydimethyl siloxane, polyurethane, polyethyl vinyl acetate, polybutylene terephthalate, and bioabsorbable polyesters derived from cyclic monomers selected from the group consisting of lactide, glycolide, epsilon-caprolactone, para-dioxanone, and trimethylene carbonate; the amphiphilic copolymeric matrix comprises polyethylene glycol; the amphiphilic copolymeric matrix comprises polyethylene glycol and at least one other polymer segment selected from polydimethyl siloxane, polyurethane, polyethyl vinyl acetate, polybutylene terephthalate, and bioabsorbable polyesters derived from cyclic monomers selected from the group consisting of lactide, glycolide, epsilon-caprolactone, para-dioxanone, and trimethylene carbonate; the amphiphilic copolymeric matrix comprises a block copolymer comprising polyethylene glycol segments and polydimethyl siloxane segments; the amphiphilic copolymeric matrix comprises a block copolymer comprising polyethylene glycol segments and polyurethane segments, where in further optional embodiments: the polyurethane segments are essentially nonabsorbable, or the polyurethane segments contain are bioabsorbable, or the polyurethane segments are synthesized from monomers that are chemically modified forms of tyrosine (Bezwada's polyurethanes); the amphiphilic copolymeric matrix comprises a block copolymer comprising a polyethylene glycol segment and a polybutylene terephthalate segment; the amphiphilic copolymeric matrix comprises a block copolymer comprising which is a diblock copolymer; the amphiphilic copolymeric matrix comprises a block copolymer comprising which is a triblock copolymer; and the bioactive agent is a non-hormonal contraceptive.

In one aspect, the present disclosure provides an intravaginal device for the controlled release of at least one bioactive agent. The device comprises a biostable elastomeric, hydrophilic copolymeric matrix. The matrix comprises a material selected from: polyethylene glycol, copolymers containing a polyethylene glycol segment, blends of polyethylene glycol with a second different polymer that may or may not contain polyethylene glycol segments, and combinations thereof. In optional embodiments, one or more of the following may further describe this aspect of the present disclosure: the matrix comprises an absorbable microparticulate ion-exchanging polymer which is effective to modulate the release of the bioactive agent for a pre-determined period of time at a specific biological site; the matrix swells in an aqueous environment, e.g., in the vaginal canal; the device has an initial diameter and the matrix swells to provide a swollen diameter which is at least 5% greater in diameter than the initial diameter; the device is in the form of a ring; and the composite is linear.

In one aspect, the present disclosure provides an intravaginal device for the controlled release of at least one bioactive agent. The device comprises a biocompatible elastomeric, polymeric matrix. The matrix comprises an absorbable microparticulate ion-exchanging polymer which is effective to modulate the release of the bioactive agent for a pre-determined period of time at a specific biological site. In optional embodiments, one or more of the following may further describe this aspect of the present disclosure: the polymeric matrix comprises at least one of polyurethane, polyethyl vinyl acetate, polyethylene glycol-block-polybutylene terephthalate, and bioabsorbable polyesters derived from cyclic monomers selected from the group consisting of lactide, glycolide, epsilon-caprolactone, para-dioxanone, and trimethylene carbonate, and combinations or blends thereof; the device comprises a polyurethane which is essentially nonabsorbable; the device comprises a polyurethane which is nonabsorbable; the device comprises a polyurethane which is essentially bioabsorbable; the device comprises a polyurethane which is bioabsorbable; the device comprises a polyurethane which is synthesized from monomers that are chemically modified forms of tyrosine (Bezwada's polyurethanes); the device comprises a block copolymer comprising at least one of a polyethylene glycol segment and a polybutylene terephthalate segment; the device comprises a diblock copolymer; the device comprises a triblock copolymer; the device is in the shape of a ring; and the bioactive agent is a non-hormonal contraceptive.

As defined by the claims, the present invention provides an intravaginal device for the controlled release of at least one bioactive agent, wherein the device has the form of a circular mesh with a polymeric ring around the perimeter of the mesh. The at least one bioactive agent is capable of 1) achieving contraception, 2) preventing the transmission of HIV, 3) treating bacterial infections, and/or 4) treating fungal infections.

The present invention provides an intravaginal device for the controlled release of at least one bioactive agent, wherein the device has the form of a circular film-derived mesh with a polymeric ring around the perimeter of the mesh. The at least one bioactive agent is capable of 1) achieving contraception, 2) preventing the transmission of HIV, 3) treating bacterial infections, and/or 4) treating fungal infections.

The present disclosure provides an intravaginal device for the controlled release of at least one bioactive agent, wherein the device has the form of a circular mesh with a polymeric ring around the perimeter of the mesh. The mesh comprises at least one bioactive agent that is optionally capable of 1) achieving contraception, 2) preventing the transmission of HIV, 3) treating bacterial infections, and/or 4) treating fungal infections.

In one aspect, the present disclosure provides an intravaginal device for the controlled release of at least one bioactive agent, wherein the device has the form of a circular fibrous mesh with a polymeric ring around the perimeter of the mesh. The mesh is a non-woven, electrospun construct. Optionally, the at least one bioactive agent is capable of 1) achieving contraception, 2) preventing the transmission of HIV, 3) treating bacterial infections, and/or 4) treating fungal infections.

In one aspect, the present invention provides an intravaginal device for the controlled release of at least one bioactive agent, wherein the device has the form of a circular fibrous mesh with a polymeric ring around the perimeter of the mesh. The mesh is a non-woven, electrospun construct, and the ring comprises at least one bioactive agent that is optionally capable of 1) achieving contraception, 2) preventing the transmission of HIV, 3) treating bacterial infections, and/or 4) treating fungal infections.

In one aspect, the present disclosure provide an intravaginal device for the controlled release of at least one bioactive agent, wherein the device has the form of a circular fibrous mesh with a polymeric ring around the perimeter of the mesh. The mesh is a non-woven, electrospun construct. The ring and mesh both contain at least one bioactive agent that is optionally capable of 1) achieving contraception, 2) preventing the transmission of HIV, 3) treating bacterial infections, and/or 4) treating fungal infections.

In one aspect, the present disclosure provides an intravaginal device for the controlled release of at least one bioactive agent, wherein the device has the form of a circular fibrous mesh with a polymeric ring around the perimeter of the mesh. The mesh is a non-woven, electrospun construct. The ring contains at least one bioactive agent that is optionally capable of 1) achieving contraception and/or 2) preventing the transmission of HIV, and wherein the mesh contains at least one bioactive agent that is capable of treating vaginal infections.

In one aspect, the present invention provides an intravaginal device for the controlled release of at least one bioactive agent, wherein the device has the form of a circular fibrous mesh with a polymeric ring around the perimeter of the mesh. The mesh has a pore size less than 100 microns. Optionally, the at least one bioactive agent that is capable of 1) achieving contraception, 2) preventing the transmission of HIV, 3) treating bacterial infections, and/or 4) treating fungal infections.

In one aspect, the present disclosure provides an intravaginal device for the controlled release of at least one bioactive agent, wherein the device has the form of a circular fibrous mesh with a polymeric ring around the perimeter of the mesh. The mesh has a pore size greater than 100 microns. Optionally, the at least one bioactive agent that is capable of 1) achieving contraception, 2) preventing the transmission of HIV, 3) treating bacterial infections, and/or 4) treating fungal infections.

In one aspect, the present disclosure provides an intravaginal device for the controlled release of at least one bioactive agent, wherein the device has the form of a circular fibrous mesh construct with a polymeric ring around the perimeter of the mesh construct. The mesh construct comprises a woven fibrous mesh covered with a non-woven electrospun mesh on the exterior of the woven fibrous mesh. Optionally, the at least one bioactive agent is capable of 1) achieving contraception, 2) preventing the transmission of HIV, 3) treating bacterial infections, and/or 4) treating fungal infections.

In one aspect, the present disclosure provides an intravaginal device for the controlled release of at least one bioactive agent, wherein the device has the form of a circular fibrous mesh construct with a polymeric ring around the perimeter of the mesh construct. The mesh construct comprises a woven copper mesh. Optionally, the at least one bioactive agent is capable of 1) achieving contraception, 2) preventing the transmission of HIV, 3) treating bacterial infections, and/or 4) treating fungal infections.

In one aspect, the present disclosure provides an intravaginal device for the controlled release of at least one bioactive agent, wherein the device has the form of a circular fibrous mesh construct with a polymeric ring around the perimeter of the mesh construct. The mesh construct comprises a woven copper mesh covered with a non-woven electrospun mesh on the exterior of the woven copper mesh. Optionally, the at least one bioactive agent is capable of 1) achieving contraception, 2) preventing the transmission of HIV, 3) treating bacterial infections, and/or 4) treating fungal infections.

In one aspect, the present disclosure provides an intravaginal device for the controlled release of at least one bioactive agent, wherein the device has the form of a circular fibrous mesh construct with a polymeric ring around the perimeter of the mesh construct. The mesh construct comprises a woven copper mesh covered with a non-woven, electrospun bioabsorbable mesh on the exterior of the woven copper mesh. Optionally, the at least one bioactive agent is capable of 1) achieving contraception, 2) preventing the transmission of HIV, 3) treating bacterial infections, and/or 4) treating fungal infections.

In one aspect, the present disclosure provides a metal-ion eluting intravaginal device for the controlled release of at least one bioactive agent. The device has the form of a circular fibrous mesh construct with a polymeric ring around the perimeter of the mesh construct. The mesh construct comprises metal ion-impregnated fibers. Optionally, the at least one bioactive agent is capable of 1) achieving contraception, 2) preventing the transmission of HIV, 3) treating bacterial infections, and/or 4) treating fungal infections.

In one aspect, not according to the invention, the present disclosure provides a fiber-reinforced intravaginal device for the controlled release of at least one bioactive agent. The device comprises a polymeric matrix in the shape of a ring, wherein fibers are dispersed throughout the matrix. Optionally, the at least one bioactive agent is capable of 1) achieving contraception, 2) preventing the transmission of HIV, 3) treating bacterial infections, and/or 4) treating fungal infections. In optional embodiments, one or more of the following may further describe this aspect of the present disclosure: the fibers are absorbable; the fibers are absorbable fibers and are drug-releasing; the fibers are absorbable and provide a controlled release of at least one bioactive agent.

In one aspect, not according to the invention, the present disclosure provides a fiber-reinforced intravaginal device for the controlled release of at least one bioactive agent. The device comprises a polymeric matrix in the shape of a ring, wherein the polymeric matrix is reinforced by a fiber made from a material selected from the group comprising nitinol, copper, titanium, Teflon, polyethylene, and polyethylene terephthalate. Optionally, the at least one bioactive agent is capable of 1) achieving contraception, 2) preventing the transmission of HIV, 3) treating bacterial infections, and/or 4) treating fungal infections.

In one aspect, not according to the invention, the present disclosure provides a fiber-reinforced intravaginal device for the controlled release of at least one bioactive agent. The device comprises a polymeric matrix in the shape of a ring, wherein the polymeric matrix is reinforced by a circular fiber that is fully embedded within the matrix. The circular fiber provides structural integrity. Optionally, the at least one bioactive agent is capable of 1) achieving contraception, 2) preventing the transmission of HIV, 3) treating bacterial infections, and/or 4) treating fungal infections.

In one aspect, the present disclosure provides an intravaginal device for the controlled release of at least one bioactive agent. The devices comprises a biostable, elastomeric polymeric matrix, wherein the matrix provides multiple phases of drug release selected from an immediate burst release, a delayed intermediate release, and a sustained long-term release. In optional embodiments, one or more of the following may further describe this aspect of the present disclosure: the matrix is in the shape of a ring; the matrix comprises an absorbable microparticulate ion-exchanging polymer to modulate the release of the bioactive agent for a pre-determined period of time at a specific biological site; the sustained long-term release is delayed; the delayed intermediate release is the result of erosion of the matrix; the immediate burst release is the result of bioactive agent being released from the surface of the device.

In one aspect, the present disclosure provides an intravaginal device for the controlled release of at least one bioactive agent. The device comprises a biostable, elastomeric polymeric matrix, wherein the matrix is in the shape of a ring. Optionally, the matrix comprises an absorbable microparticulate ion-exchanging polymer to modulate the release of the bioactive agent for a pre-determined period of time at a specific biological site. The controlled release of the at least one bioactive agent occurs by one or more of the following three mechanisms, the three mechanisms comprising: (a) surface release of drug; (b) erosion of the polymeric matrix; (c) diffusion of drug through the matrix. The surface release of drug results in an immediate burst effect. The erosion of the polymeric matrix results in a delayed intermediate release of drug and/or bioactive agent. The diffusion of drug from the matrix results in the sustained release of drug and/or bioactive agent.

In another aspect, the present disclosure provides an intravaginal device for the controlled release of at least one bioactive agent. The device comprises a biostable, elastomeric polymeric matrix which is in the form of a ring. The matrix comprises an absorbable microparticulate ion-exchanging polymer to modulate the release of the bioactive agent for a desired period of time at a specific biological site. The matrix provides at least one mechanism of bioactive agent release selected from: (a) surface release from the device that creates an immediate burst release effect, (b) erosion of the polymeric matrix that creates a delayed intermediate release effect, and (c) diffusion of bioactive agent through the polymeric matrix that creates a sustained, long-term release effect. The dosage of the at least one bioactive agent depends upon the predominating mechanism of bioactive agent release at any given point in time.

In one aspect, the present disclosure provides an intravaginal ring for the controlled release of at least one bioactive agent. The ring comprises a bioactive agent agent-eluting polymeric matrix. At least one bioactive agent is a probiotic strain. For example, the probiotic strain may be a strain of Lactobacillus.

In one aspect, the present disclosure provides an intravaginal ring for the controlled release of at least one bioactive agent. The ring comprises a bioactive agent-eluting polymeric matrix. At least one bioactive agent is contained within nanoparticles, where the nanoparticles are dispersed throughout the polymeric matrix.

In one aspect, the present disclosure provides an intravaginal ring for the controlled release of at least one bioactive agent. The intravaginal ring comprises a biostable, elastomeric polymeric matrix. At least one bioactive agent is a non-hormonal contraceptive in the form of ferrous ascorbate.

In one aspect, the present disclosure provides an intravaginal ring for the controlled release of at least one bioactive agent. The intravaginal ring comprises a biostable, elastomeric polymeric matrix. At least one bioactive agent is a non-hormonal contraceptive in the form of an organometallic complex, wherein the organometallic complex provides at least one metal ion capable of acting as a reducing agent.

In one aspect, the present disclosure provides an intravaginal ring for the controlled release of a non-hormonal contraceptive bioactive agent and a second bioactive agent for the prevention of HIV transmission. The intravaginal ring comprising a biostable, elastomeric polymeric matrix. The second bioactive agent for the treatment of HIV transmission may be tenofovir. The matrix may comprise an absorbable microparticulate ion-exchanging polymer to modulate the release of the bioactive agent(s) for a pre-determined period of time at a specific biological site.

In one aspect, the present disclosure provides an intravaginal ring for the controlled release of at least one bioactive agent effective for contraception. The intravaginal ring comprises a polymeric matrix. At least one bioactive agent is a reducing agent capable of reducing the oxidation state of at least one other bioactive agent, wherein the at least one other bioactive agent provides a spermiostatic effect.

Devices as identified herein achieve the controlled release of at least one bioactive agent by way of incorporating into the device one or more of: a non-bioabsorbable microparticulate ion-exchanging polymer, a fully bioabsorbable polymeric matrix, a biostable hydrophilic elastomeric polymeric matrix, a biostable amphiphilic elastomeric polymeric matrix, a biostable elastomeric polymeric matrix containing an inorganic microparticulate, and a biostable elastomeric porous polymeric matrix. When present, each of these components may be present in the device at a weight percent (weight of component divided by weight of device, times 100) of at least 5%, or at least 10%, or at least 15%, or at least 20%, or at least 25%, or at least 30%, or at least 35%, or at least 40%, or at least 45%, or at least 50%, or at least 55%, or at least 60%, or at least 65%, or at least 70%, or at least 75%, or at least 80%, or at least 85%, or at least 90%, or at least 95%.

The devices of the present disclosure provide various advantages. For example, an advantage of incorporating an essentially non-absorbable microparticulate into a device is that the microparticulate remains intact and dispersed throughout the polymeric matrix over the predetermined time frame that the device is implanted. Such microparticulates can be used to modulate the polarity of the matrix, thereby affecting solubility, diffusion and release of bioactive agents from the matrix. Microparticles bearing polar (e.g., carboxylic acid, amine, hydroxyl) or nonpolar functional groups (alkyl, benzyl) at the surface can be dispersed throughout the matrix. Microparticles with contrasting polarity relative to the matrix polymer can be utilized to create domains wherein a bioactive agent or agents can be localized. This provides for an additional mechanism for controlling release of bioactive agents from the matrix. That is, bioactive agents must first dissociate from the microparticlulate domains, and then diffuse to the matrix surface where said agents are released into the local media. Other bioactive agents can be dispersed within the matrix, outside of the microparticulate domains, and be released strictly by diffusion through the matrix and release from the surface. In the instances where microparticulates are of relatively similar polarity to the polymer matrix, the microparticles can be at least partially soluble in the matrix to create one homogeneous phase.

Fully absorbable polymeric matrices provide an alternative mechanism for drug release from the polymeric matrix. The bioabsorbable matrix is designed to degrade, typically via hydrolysis, releasing bioactive agents in a time-dependent manner that depends upon the rate of degradation. Furthermore, the composition of the absorbable matrix can be tailored to degrade over a predetermined time frame.

The hydrophilic polymeric matrices provide a functional advantage that relates directly to the solubility of bioactive agents within the matrix, as well as absorption of water from the local aqueous environment. An intravaginal rings that is composed of a hydrophobic, elastomeric, polydimethylsiloxane-based matrix may be effective in solubilizing nonpolar drugs and bioactive agents; however, these matrices are not as effective at solubilizing polar additives and bioactive agents, and for this reason, hydrophilic matrices are desirable alternatives.

An amphiphilic copolymeric matrix provides both polar and nonpolar phases within the same drug-releasing matrix. This phase separation advantageously allows for the segregation of bioactive agents into different portions of the matrix. Furthermore, bioactive agents of different polarities can be incorporated into the construct with less complications relating to drug-matrix compatability.

The incorporation of inorganic microparticulates or salts into the ring matrix can be used to modulate the release of bioactive agents. The microparticulates or salts can be selected to increase or decrease water absorption into the matrix, and thereby affect the swelling of the matrix. This in turn directly affects the rate of diffusion of water into the matrix and the diffusion of bioactive agents out of the matrix. Examples of suitable inorganic microparticulates or salts can be selected from the oxides, carbonates, sulfates, and halides.

Copolymeric matrices that become microporous as the microparticulate degrades over a predetermined time frame allow for an increase in porosity over time. The development of porosity throughout the matrix can be designed to coincide with the exhaustion of bioactive agents that are present within the matrix, thereby facilitating the release of the bioactive agent which remains. Thus, during the final phase of implantation this transition in porosity ensures that the effective concentrations of bioactive agent continue to be released. The degradation of the microparticulate, and the corresponding increase in porosity, can be tailored to coincide with the most fertile period in the menstrual cycle such that the release of bioactive agents occurs when contraception is most needed.

Porous matrices are beneficial alternatives to non-porous matrices. Porosity allows for water to flow into the interior of the polymeric matrix, which facilitates the absorption of water and the release of bioactive agents. An open-pore structure also allows for the release of bioactive agents that might be trapped deep within the matrix.

Multilayered intravaginal rings provide for alternative constructions over traditional intravaginal rings. One advantage is that multiple layers can be designed to contain specific concentrations of bioactive agents within each layer. This type of construction can be used to control dosing of a bioactive agent or agents over a predetermined time frame. For instance, the most exterior layer of the ring may contain a bioactive agent intended to be released quickly - either immediately as in a burst release or over a period of hours or days. An intermediate layer could contain the same or different bioactive agent, or a combination thereof, and provide a phase of intermediate release of said bioactive agent or bioactive agents. If present, a deeper more interior layer can provide for a delayed release that allows for a sustained release of a bioactive agent or bioactive agents. Furthermore, the concentration of bioactive agent can be varied in each layer to create an appropriate dosing effect. Concentrations can be decreased in the outermost layer, for instance, in order to reduce the burst effect that is often observed when intravaginal rings are first implanted. Furthermore, concentrations within the more interior layers can be increased to either maintain a sustained release over a predetermined time frame or to provide a delayed release of a particular bioactive agent or combination of bioactive agents at increased levels. Another advantage of a multilayered construction is the ability to segregate bioactive agents to particular layers of the device. This separation of bioactive agents can allow for the control of drug dosing and release kinetics that would be difficult to obtain from an intravaginal ring that contains drug evenly dispersed throughout the matrix.

Furthermore, each layer of a multi-layer device can be designed or fabricated from absorbable or nonabsorbable polymeric components. One benefit of having absorbable layers is that the exterior surface of an intravaginal ring can be hydrolyzed over time and release at least one bioactive agent in a short time frame. An example of an absorbable/nonabsorbable multilayered intravaginal ring is a device wherein the outer layer is absorbable (i.e. fabricated from a hydrolysable polyester). This outer layer is degraded by hydrolysis in an aqueous environment, which results in the release of the bioactive agent(s) that are dispersed within this layer. Once the outer layer is hydrolyzed, secondary release of bioactive agents from the underlying layer can occur, potentially by diffusion to provide sustained release or delayed release of the same or a different bioactive agent.

Thus, the present devices which incorporate one or more of an essentially non-absorbable microparticulate, a fully bioabsorbable polymeric matrix, a biostable, hydrophilic elastomeric polymeric matrix, a biostable, amphiphilic elastomeric polymeric matrix, a matrix that incorporates inorganic microparticulates, a microporous polymeric matrix, a biostable, elastomeric porous polymeric matrix, and a biostable, elastomeric copolymeric matrix, any of which comprising multiple layers, provide various advantages as described herein.

The Examples provided below further illustrate and exemplify the devices of the present invention and methods of preparing such devices. The starting materials and various reactants utilized or referenced in the examples may be obtained from commercial sources, or are readily prepared from commercially available organic compounds, using methods well-known to one skilled in the art. For example, the devices of the present invention may be prepared and used in analogy to the methods and uses provided in U.S. Patent Nos. 8057817 and 8404272. See also, US. Patent Nos. 7910126; 83236679; 8580293; 8580294; US Publication Nos. 2012/0053534; 2012/0097171; 2012/0177716; 2013/0042873.

### Comparative EXAMPLES Example 1

An intravaginal ring for the controlled release of at least one bioactive agent, where the ring includes a biostable elastomeric polymeric matrix, and the matrix includes an essentially non-bioabsorbable microparticulate ion-exchanging polymer that is effective to modulate the release of the bioactive agent from the ring, is prepared as follows.

A ring is synthesized by physically mixing a two-component biomedical-grade silicone (available from, for example, Dow-Corning in Midland, MI USA under their SILASTIC tradename; or from Master Bond Inc. in Hackensack, NJ USA or Bluestar Silicones in East Brunswick, NJ USA) with ferrous gluconate (FG), ascorbic acid, glycine, and polyacrylic acid microparticles (available from, for example, Sigma-Aldrich, St. Louis, MI USA). The mixture is injected into a cavity mold, which is subsequently heated to 80°C until the two-part silicone cures. The mold cavity is shaped in the form of a ring with an outside diameter of 55 mm and an inside diameter of 40.0 mmm to provide a tubular ring material having a diameter of about 15 mm.

### Example 2

An intravaginal ring for the controlled release of at least one bioactive agent, where the ring includes a fully-bioabsorbable polymeric matrix, and the matrix includes an absorbable microparticulate ion-exchanging polymer that is effective to modulate release of the bioactive agent from the ring, is prepared as follows.

A ring is synthesized by blending particles of an aliphatic, triaxial copolyester (available from, e.g., Poly-Med, Anderson, SC USA under their STRATAPRENE tradename) with ferrous gluconate (FG), ascorbic acid (AA), glycine, and polyglycolide microparticles (available from, for example, Poly-Med, Anderson, SC USA). The polyester particles have a diameter of less than 4 mm, or a diameter in the range of from 0.5 to 4.0 mm. The blended mixture is injected into a cavity mold and heated to 120°C until the polyester particles have melted and taken on the shape of the cavity. The mold cavity is ring-shaped with an outside diameter of 55 mm and an inside diameter of 40.0 mm.

### Example 3

An intravaginal ring for the controlled release of at least one bioactive agent, where the ring includes a biostable hydrophilic elastomeric polymeric matrix, and the matrix includes an absorbable microparticulate ion-exchanging polymer that is effective to modulate release of the bioactive agent from the ring, is prepared as follows.

A ring is formed by physically mixing a polyether urethane urea (PEUU) with ferrous gluconate (FG), ascorbic acid (AA), glycine, and polyglycolide microparticles. The mixture is injected into a cavity mold which is heated to 120°C until the polymer-additive blend fills the cavity, which is shaped in a ring with an outside diameter of 55 mm and an inside diameter of 40.0 mm. The PEUU is initially prepared (before blending with the other ring components) by synthesizing a hydrophilic polyether urethane prepolymer, followed by reacting with a diamine to create the PEUU. The polymer is then ground into smaller particles suitable for injection molding (particles with a diameter < 4 mm).

### Example 4

An intravaginal ring for the controlled release of at least one bioactive agent, where the ring includes a biostable amphiphilic elastomeric polymeric matrix, and the matrix includes an absorbable microparticulate ion-exchanging polymer effective to modulate release of the bioactive agent from the ring, may be prepared as follows.

A ring is synthesized by physically mixing a reactive two-part silicone where one part is a hydrophilic silicone (available from, for example, Gelest, Inc., headquartered in Morrisville, PA, USA; see, e.g., dimethylsiloxane-ethylene oxide block/graft copolymers) and the second part is a hydrophobic reactive silicone with Si-OH end groups, with ferrous gluconate (FG), ascorbic acid (AA), glycine, and polyglycolide microparticles. The mixture is injected into a cavity mold, which is subsequently heated to 200°C until the two-part silicone cures. The mold cavity is shaped in the form of a ring with an outside diameter of 55 mm and an inside diameter of 40.0 mm.

### Example 5

An intravaginal ring for the controlled release of at least one bioactive agent, where the ring includes a biostable amphiphilic elastomeric polymeric matrix, and the matrix includes an absorbable microparticulate ion-exchanging polymer effective to modulate release of the bioactive agent from the ring, is prepared as follows.

A ring is synthesized by physically mixing a two-part biomedical-grade polyester silicone (available from, e.g., Evonik, Essen, North Rhine-Westphalia, Germany under the SILKOFTAL tradename) with ferrous gluconate (FG), ascorbic acid (AA), glycine, and polyglycolide microparticles. The mixture is injected into a cavity mold, which is subsequently heated to 200°C until the two-part silicone cures. The mold cavity is shaped in the form of a ring with an outside diameter of 55 mm and an inside diameter of 40.0 mm.

### Example 6

An intravaginal ring for the controlled release of at least one bioactive agent, where the ring includes a biostable amphiphilic elastomeric polymeric matrix, and the matrix includes an absorbable microparticulate ion-exchanging polymer effective to modulate release of the bioactive agent from the ring, is prepared as follows.

A ring is synthesized by physically mixing an amphiphilic silicone (available from, for example, Gelest, Inc. of Morrisville, PA, USA; see, e.g., dodecyl methylsiloxane-hydroxypolyalkyleneoxypropyl methylsiloxane, copolymer, CAS No. 145686-74-4) with ferrous gluconate (FG), ascorbic acid (AA), glycine, and polyglycolide microparticles. The mixture is injected into a cavity mold, which is subsequently heated to 250°C until the mixture forms the desired shape. The mold cavity is shaped in the form of a ring with an outside diameter of 55 mm and an inside diameter of 40.0 mm.

## Claims

1. An intravaginal ring for the controlled release of at least one bioactive agent, the ring comprising
a) a biostable elastomeric porous polymeric matrix being a silicone-based polymer,
b) an absorbable polymeric microparticulate effective to modulate release of the bioactive agent from the ring, and
c) a circular mesh, wherein the matrix is in the shape of a ring around the perimeter of the mesh;
where the matrix becomes microporous as the microparticulate degrades when exposed to bodily fluid; and wherein the intravaginal ring is not fiber reinforced.

2. The intravaginal ring of claim 1, wherein the at least one bioactive agent is a non-hormonal bioactive agent.

3. The intravaginal ring of claim 2, wherein the non-hormonal bioactive agent is a non-hormonal contraceptive in the form of ferrous gluconate.

4. The intravaginal ring of any of claims 1 to 3, wherein the biostable elastomeric porous polymeric matrix is non-absorbable.

5. The intravaginal ring of any of claims 1 to 4, wherein the microparticles have an average diameter in the range 0.5 µm to 100 µm, preferably 10 µm to 80 µm and more preferably 20 µm to 70 µm.

6. The intravaginal ring of any of claims 1 to 5, wherein the circular mesh has a pore size of less than 100 microns or of greater than 100 microns.

7. The intravaginal ring of any of claims 1 to 6, wherein the absorbable polymeric microparticulate is not ion-exchanging.

8. The intravaginal ring of claim 7, wherein the not ion-exchanging microparticulate is prepared by initiating ring-opening polymerization of cyclic monomers selected from the group consisting of lactides, glycolide, epsilon-caprolactone, trimethylene carbonate, and para-dioxanone, and combinations thereof with an alcohol, such as 1,3-propane diol or decyl alcohol.

## Patentansprüche

1. Intravaginalring zur kontrollierten Freisetzung mindestens eines bioaktiven Wirkstoffs, wobei der Ring
a) eine biostabile, elastomere, poröse Polymermatrix, die ein Polymer auf Silikonbasis ist,
b) ein resorbierbares polymeres Mikropartikelmaterial, das dazu geeignet ist, die Freisetzung des bioaktiven Wirkstoffs aus dem Ring zu modulieren, und
c) ein kreisförmiges Netz, wobei die Matrix entlang des Umfangs des Netzes ringförmig ausgebildet ist, umfasst,
wobei die Matrix infolge des Abbaus des Mikropartikelmaterials bei Einwirkung von Körperflüssigkeit mikroporös wird und wobei der Intravaginalring nicht faserverstärkt ist.

2. Intravaginalring nach Anspruch 1, wobei der mindestens eine bioaktive Wirkstoff ein nichthormoneller bioaktiver Wirkstoff ist.

3. Intravaginalring nach Anspruch 2, wobei der nichthormonelle bioaktive Wirkstoff ein nichthormonelles Kontrazeptivum in Form von Eisengluconat ist.

4. Intravaginalring nach einem der Ansprüche 1 bis 3, wobei die biostabile, elastomere, poröse Polymermatrix nicht resorbierbar ist.

5. Intravaginalring nach einem der Ansprüche 1 bis 4, wobei die Mikropartikel einen mittleren Durchmesser im Bereich von 0,5 µm bis 100 µm, vorzugsweise von 10 µm bis 80 µm und besonders bevorzugt von 20 µm bis 70 µm aufweisen.

6. Intravaginalring nach einem der Ansprüche 1 bis 5, wobei das kreisförmige Netz eine Porengröße von weniger als 100 µm oder von mehr als 100 µm aufweist.

7. Intravaginalring nach einem der Ansprüche 1 bis 6, wobei das resorbierbare polymere Mikropartikelmaterial nicht ionenaustauschend ist.

8. Intravaginalring nach Anspruch 7, wobei das nicht ionenaustauschende Mikropartikelmaterial durch Initiierung einer Ringöffnungspolymerisation zyklischer Monomere, die aus der Gruppe bestehend aus Lactiden, Glycolid, ε-Caprolacton, Trimethylencarbonat und para-Dioxanon sowie Kombinationen hiervon ausgewählt sind, in Gegenwart eines Alkohols, beispielsweise 1,3-Propandiol oder Decylalkohol, hergestellt wird.

## Revendications

1. Anneau intravaginal pour libération régulée d'au moins un agent bioactif, lequel anneau comprend :
a) une matrice de polymère élastomère, poreuse et biostable, qui est en un polymère à base de silicone,
b) un ensemble de microparticules de polymère résorbables, qui ont pour effet de moduler la libération de l'agent bioactif à partir de l'anneau,
c) et un maillage circulaire, étant entendu que la matrice a la forme d'un anneau placé sur le pourtour du maillage,
dans lequel la matrice devient microporeuse à mesure que les microparticules se dégradent lors de leur exposition aux fluides corporels, et lequel anneau intravaginal n'est pas renforcé de fibres.

2. Anneau intravaginal conforme à la revendication 1, dans lequel l'agent bioactif, au nombre d'au moins un, est un agent bioactif non hormonal.

3. Anneau intravaginal conforme à la revendication 2, dans lequel l'agent bioactif non hormonal est un contraceptif non hormonal sous forme de gluconate ferreux.

4. Anneau intravaginal conforme à l'une des revendications 1 à 3, dans lequel la matrice de polymère élastomère poreuse et biostable n'est pas résorbable.

5. Anneau intravaginal conforme à l'une des revendications 1 à 4, dans lequel les microparticules présentent un diamètre moyen qui se situe dans l'intervalle allant de 0,5 µm à 100 µm, de préférence de 10 µm à 80 µm, et mieux encore de 20 µm à 70 µm.

6. Anneau intravaginal conforme à l'une des revendications 1 à 5, dans lequel le maillage circulaire présente des pores de taille inférieure à 100 microns ou supérieure à 100 microns.

7. Anneau intravaginal conforme à l'une des revendications 1 à 6, dans lequel l'ensemble de microparticules de polymère résorbables n'est pas un échangeur d'ions.

8. Anneau intravaginal conforme à la revendication 7, pour lequel on a préparé l'ensemble de microparticules non échangeur d'ions en amorçant une polymérisation avec ouverture de cycle de monomères cycliques, choisis dans l'ensemble formé par les lactides, glycolide, epsilon-caprolactone, carbonate de triméthylène et para-dioxanone et leurs combinaisons, avec un alcool, tel que le propane-1,3-diol ou l'alcool décylique.
